# EUROPEAN PATENT APPLICATION

(11) **EP 3 614 142 A1**
(43) Date of publication of application: **26.02.2020**
(21) Application number: 18190489.7
(22) Date of filing: 23.08.2018
(51) Int. Cl.: G01N 33/00

(54) **DETERMINING THE EMISSION LOCATION OF A SUBSTANCE**

(71) Applicant: Rain Carbon GmbH, 44579 Castrop-Rauxel (DE)
(72) Inventor: D'HONDT, Bram Sebastiaan Kristiaan, 3960 Bree (BE)
(74) Representative: Cohausz & Florack

(57) **Abstract**

A method is disclosed comprising: gathering an environmental information indicative of a sense-able substance; determining a location estimate information indicative of an estimation of a location of an emission of the substance; determining a controlling information indicative of a movement of the at least one apparatus towards the estimation of the location of the sense-able substance, wherein the controlling information is determined based at least partially on the location estimate information; controlling the at least one apparatus towards the source of the sense-able substance based at least partially on the controlling information; determining a position information indicative of the location of the emission of the substance in case the location of the emission of the substance is reached; and outputting the position information. Further disclosed are a corresponding apparatus, and a corresponding system.

## Description

### FIELD

The invention relates to the field of determining locations of substances, or more particularly relates to determining the location of an emission of a sense-able substance.

### BACKGROUND

Exploration of the use of Unmanned Aerial Vehicles (UAV), e.g. drones by industry (e.g. several applications like delivery of goods and products, carrying equipment, monitoring certain area, to name but a few non-limiting examples) is rapidly expanding.

There is an interest in determining whether or not an emission of certain substances that e.g. are stored for further purposes, takes place. Such an emission can happen e.g. when a storage containing the substances is erroneous. Further, the emission of certain substances may contain a certain risk, e.g. ranging from the risk of inflaming to the risk of persons inhaling the emission of the substance that might be harmful for persons. For example, in plants, some products can be dangerous or harmful for humans and/or the environment. Those products can be transported in pipes and be stored in vessels. Further, the products should be kept inside the pipes and vessels. Due to e.g. corrosion, untighten flanges or other errors, emissions to the soil and atmosphere can occur. Currently sites comprising such pipes and vessels are equipped with standard, static volatile organic compounds (VOC) measurements. These measurements indicate if a leak is active. Depending e.g. on the wind direction, a different measurement may detect the leak, which can lead to erroneous indications of the location of the leak. Therefore, manual search to locate the source of the leak is required.

It is known to manually determine locations of such emissions, e.g. by persons equipped with detectors searching for locations of such emissions. Meaning one person has to go, e.g. outside where the substances are stored. The persons use a portable detector and search for the location respectively source of the emission by doing many measurements with the detector approximating the source of the emission. This process is time consuming. Further, some of those emissions may be non-continuous so that it might not be possible at all to determine the location of the emission of such substances due to the time it may take.

### SUMMARY OF SOME EXAMPLE EMBODIMENTS OF THE INVENTION

It is thus, inter alia, an object of the present invention to provide a solution in which a determining of a location of an emission of a substance can be performed in a reliable and efficient manner.

According to a first exemplary aspect of the present invention, a method is disclosed, the method comprising:
- gathering an environmental information indicative of a sense-able substance;
- determining a location estimate information indicative of an estimation of a location of an emission of the substance;
- determining a controlling information indicative of a movement of at least one apparatus towards the estimation of the location of the sense-able substance, wherein the controlling information is determined based at least partially on the location estimate information;
- controlling the at least one apparatus towards the source of the sense-able substance based at least partially on the controlling information;
- determining a position information indicative of the location of the emission of the substance in case the location of the emission of the substance is reached; and
- outputting the position information.

This method may for instance be performed and/or controlled by an apparatus, for instance a drone.

According to a further exemplary aspect of the invention, a computer program is disclosed, the computer program when executed by a processor causing an apparatus, for instance a drone, to perform and/or control the actions of the method according to the first exemplary aspect.

The computer program may be stored on computer-readable storage medium, in particular a tangible and/or non-transitory medium. The computer readable storage medium could for example be a disk or a memory or the like. The computer program could be stored in the computer readable storage medium in the form of instructions encoding the computer-readable storage medium. The computer readable storage medium may be intended for taking part in the operation of a device, like an internal or external memory, for instance a Read-Only Memory (ROM) or hard disk of a computer, or be intended for distribution of the program, like an optical disc.

According to a further exemplary aspect of the invention, an apparatus is disclosed, configured to perform and/or control or comprising respective means for performing and/or controlling the method according to the first exemplary aspect.

The means of the apparatus can be implemented in hardware and/or software. They may comprise for instance at least one processor for executing computer program code for performing the required functions, at least one memory storing the program code, or both. Alternatively, they could comprise for instance circuitry that is designed to implement the required functions, for instance implemented in a chipset or a chip, like an integrated circuit. In general, the means may comprise for instance one or more processing means or processors.

According to a further exemplary aspect of the invention, an apparatus is disclosed, comprising at least one processor and at least one memory including computer program code, the at least one memory and the computer program code configured to, with the at least one processor, cause an apparatus, for instance the apparatus, at least to perform and/or to control the method according to the first exemplary aspect.

The above-disclosed apparatus according to any aspect of the invention may be a module or a component for a device, for example a chip. Alternatively, the disclosed apparatus according to any aspect of the invention may be a device, for instance be comprised by a drone. The disclosed apparatus according to any aspect of the invention may comprise only the disclosed components, for instance means, processor, memory, or may further comprise one or more additional components.

According to a further exemplary aspect of the invention, a system is disclosed, comprising at least one apparatus according to any aspect of the invention as disclosed above, and a server, a server-cloud (e.g. more than one apparatuses, for instance at least two servers communicating with each other over a communication network, e.g. the Internet), or a terminal, wherein the at least one apparatus and the server, the server cloud, or the terminal are configured to perform and/or control the method according to the first exemplary aspect of the present invention together.

In some exemplary embodiments of the present invention, some method steps of the method according to the first exemplary aspect of the present invention may thus be performed and/or controlled by the server, the server cloud, or the terminal (e.g. determining the location estimate information, determining the controlling information). Other method steps (e.g. gathering of the environmental information, controlling the at least one apparatus, determining the position information, outputting the position information) of the method according to the first exemplary aspect of the present invention may for instance be performed and/or controlled by the at least one apparatus (e.g. a drone) communicating with the server or the server cloud.

In the following, exemplary features and exemplary embodiments of all aspects of the present invention will be described in further detail.

The sense-able substance may for instance be a chemical agent. The substance may for instance be sense-able by gathering at least a part of the substance, e.g. that is volatile. The substance may for instance be volatile. Such a volatile substance may for instance be of the physical state i) solid, ii) liquid, and/or iii) gas.

Such a substance may for instance be a substance having a boiling point range in degree Celsius (°C) of <0 to 50-100; 50-100 to 240-260; and/or 240-260 to 380-400. Such a substance may for instance be a very volatile (gaseous) organic compounds (VVOC). Such a substance may for instance be a VOC. Such a substance may for instance be a semi volatile organic compounds (SVOC). Such a substance may for instance be a nitrogen oxide (NOx). Such a substance may for instance be a Sulfur oxide (SOx).

The gathering of the environmental information may for instance be a measuring and/or probing of the surrounding of the apparatus. Further, such a measured and/or probed surrounding may for instance be analyzed to determine whether or not the substance is sense-able (e.g. present in the measured and/or probed surrounding). The gathered environmental information may for instance be the result of such a measuring and/or probing of the surrounding of the at least one apparatus. The gathered environmental information may for instance represent whether or not the substance is present in such a surrounding of the apparatus.

The location estimate information indicative of the estimation of a location of the emission of the substance is determined. For instance, the location estimate information may for instance be determined at least partially based on a direction from which the substance is sense-able. Further, for instance, the location estimate information may for instance be determined at least partially based on an intensity and/or a concentration of the sense-able substance. For instance, it may be determined an intensity and/or a concentration of the sense-able substance. Such an intensity and/or such a concentration may for instance be determined based at least partially on the gathered environmental information.

Further, the determining of the location estimate information may for instance be based at least partially on more than one gathered pieces of environmental information. For instance, more than one (e.g. two) pieces of environmental information may be gathered prior to the determining of the location estimate information. Each of the more than one pieces of environmental information may for instance be gathered at different locations. For instance, the at least one apparatus may gather a first environmental information at a first location. Then, the at least one apparatus may for instance gather a second environmental information at a second location. The at least one apparatus may for instance move between the gathering of the first and the second environmental information. The location estimate information may then for instance be determined based at least partially on a comparison of the first and the second environmental information. For instance, an intensity and/or a concentration of the sense-able substance of the first and the second environmental information may be compared. In case the intensity and/or the concentration of the first environmental information is higher than the intensity and/or the concentration of the sense-able substance of the second environmental information, the location estimation information may for instance be determined to represent an estimation of the location in a direction of a (e.g. calculated) line between the location at which the first environmental information was gathered, and the location at which the second environmental information was gathered. Further, the location estimation information may for instance represent a direction along such a line from the (first or second) environmental information having a lower intensity and/or concentration of the sense-able substance than the other gathered environmental information to the (second or first) environmental information having a higher intensity and/or concentration of the sense-able substance.

Further, the location estimation information may be determined based at least partially on a ratio of an intensity and/or concentration of the first (or second) environmental information to the second (or first) environmental information. Based on this ratio, for instance a distance from a current location of the at least one apparatus to the location of the emission of the sense-able substance may for instance be determined (e.g. estimated). For instance, to estimate such a distance, one or more reference values may for instance enable the determining of such a distance.

Such one or more reference values may for instance be comprised by a memory, e.g. a database. Such one or more reference values may for instance be indicative of a ratio between two intensities and/or concentrations that are associated with a certain sense-able substance. Based on e.g. the absolute value of such a ratio, a corresponding distance may for instance be obtained (e.g. from the database). For instance, a value of the ratio (associated with a certain substance) approximating a value of 1 may be indicative of a longer distance that the at least one apparatus is away from the location of the emission of the substance than another value of the ratio (associated with a certain substance) approximating a value of 0 that may be indicative of a shorter distance that the at least one apparatus is away from the location of the emission of the substance. It will be understood that further values of ratios in between the range of 0 to 1 may be indicative of a certain distance that the at least one apparatus that determined the ratio is away from the location of the emission of the substance. There may for instance be a linear relation of the corresponding ratio that is used to determine the distance from a current location of the at least one apparatus to the location of the emission of the substance. Alternatively, some substances may for instance have a non-linear relation from a current location of the at least one apparatus to the location of the emission of the substance.

The controlling information indicative of a movement of the at least one apparatus towards the estimation of the location of the sense-able substance is determined. The controlling information is determined based at least partially on the location estimate information. For instance, the controlling information may represent a direction in which the at least one apparatus can move in order to move towards the estimation of the location of the emission of the sense-able substance. Further, the controlling information may for instance comprise or represent an estimated distance to the estimation of the location of the sense-able substance. Thus, the controlling information may for instance enable the apparatus to move towards the estimation of the location of the sense-able substance.

Then, the at least one apparatus is controlled based at least partially on the controlling information, e.g. to move the at least one apparatus towards the location that was estimated of the sense-able substance.

The position information indicative of the location of the emission of the substance in case the location of the emission of the substance is reached is determined. Whether or not the location of the emission of the substance is reached - meaning that the at least one apparatus is within the vicinity respectively proximity of the emission of the substance - may for instance be determined at least partially based on an intensity and/or a concentration of the substance. The intensity and/or the concentration of the substance may for instance be determined based on another environmental information that is gathered prior to determining the position information. The at least one apparatus may for instance be within the vicinity of the emission of the substance in case the at least one apparatus is closer than 10, 5, 4, 3, 2, or 1 m of range to the location of the emission of the substance.

The (determined) position information is then output, e.g. to a server, a server cloud, a terminal, or to another apparatus that transfers the position information to the server, the server cloud or to the terminal. The position information may for instance be output by broadcasting the position information. The position information may for instance be output via a wireless communication connection, e.g. between the apparatus and the server, or between the apparatus and the terminal.

Such a terminal may for instance be an electronic device. The electronic device may for instance be portable (e.g. weigh less than 5, 4, 3, 2, or 1 kg). The electronic device may for instance be at least temporarily (e.g. in removable form) or permanently installable in a vehicle. Such a vehicle may for instance be a car, truck, or forklift, to name but a few non-limiting examples. The electronic device may for instance comprise or be connectable to a display for displaying the position information, e.g. to guide and/or navigate a user of the electronic device to the location of the emission of the substance. The electronic device may for instance comprise or be connectable to means for outputting sound, e.g. in the form of spoken commands or information, in particular with respect to the location of the emission of the substance. The electronic device may for instance comprise or be connectable to one or more sensors for determining the electronic devices position, such as a Global Navigation Satellite System (GNSS) receiver, e.g. in the form of a Global Positioning System (GPS) receiver. Such a GPS receiver may for instance enable to guide and/or navigate the user of the electronic device to the location of the emission of the substance after the position information representing this location was determined.

According to an exemplary embodiment of all aspect of the present invention, the location of the substance is reached in case the environmental information exceeds a pre-determined or determined according to pre-defined rules threshold.

Thus, there may for instance be a pre-defined or determined according to pre-defined rules threshold. The threshold may for instance represent a value. The threshold may for instance be indicative of an intensity and/or a concentration of the substance in the surrounding of the at least one apparatus. The intensity and/or concentration may for instance be determined at least partially based on e.g. another environmental information that is gathered, e.g. as disclosed above with respect to the position information that is determined. Such pre-defined or determined according to pre-defined rules threshold may for instance be associated with a certain distance that the at least one apparatus is away from the location of the emission of the substance. For instance, the intensity and/or the concentration according to an environmental information that is gathered within the vicinity and/or proximity of the at least one apparatus may for instance be compared to e.g. one or more training cases. The one or more training cases of one or more intensities and/or concentrations associated with one or more substances at a pre-defined distance (e.g. 10, 5, 4, 3, 2, or 1 m, to name but a few non-limiting examples) from the emission of the substance may for instance be determined prior to the method according to the first exemplary aspect of the present invention. For instance, at least one training case may for instance be determined per one certain substance. Those results may for instance be stored in a memory, e.g. a database that is comprised by or be connectable to the at least one apparatus. The at least one apparatus may for instance be connectable to the memory via a (e.g. wireless) communication network (e.g. the Internet).

According to an exemplary embodiment of all aspects of the present invention, the steps of the gathering of the environmental information, the determining of a location estimate information, the determining of a controlling information, and the controlling of the at least one apparatus are repeated until the location of the substance is reached.

For instance, those method steps may be repeated steadily, or may be repeated in pre-defined or determined according to pre-defined rules time intervals. By repeating the steps of the gathering of the environmental information, the determining of a location estimate information, the determining of a controlling information, and the controlling of the at least one apparatus, the at least one apparatus may for instance control itself, and as a result of the controlling, move closer to the location of the emission of the source than it was prior the respective repetition. The steps may in particular be repeated until another environmental information that is gathered by the at least one apparatus exceeds the pre-determined or determined according to pre-defined rules threshold.

According to an exemplary embodiment of all aspects of the present invention, the method further comprising:
- treatment of the substance based at least partially on the output position information.

The treatment may for instance be performed at least partially in a manual fashion. For instance, a person on site may for instance perform one or more necessary actions respectively steps in order to reduce or stop the emission of the substance. Additionally or alternatively, the treatment may for instance be performed, at least partially, in an automatic fashion. For instance, a treatment apparatus configured to perform one or more necessary actions respectively steps in order to reduce or stop the emission of the substance may for instance perform the treatment. Such a treatment apparatus may for instance a robot comprising or being connectable to means for performing necessary one or more steps respectively actions to reduce or stop the emission of the substance.

According to an exemplary embodiment of all aspects of the present invention, the environmental information is gathered by at least one sensor that is configured at least to sense one or more VOC, NOx, and/or SOx.

The at least one sensor that is configured at least to sense VOC, NOx, and/or SOx may also be referred to as at least one air pollution sensor. Such an air pollution sensor may for instance be configured to detect and/or monitor the presence of air pollution by the sense-able substance in the surrounding area (of the at least one apparatus). The at least one sensor may for instance be configured to sense (e.g. detect) gases and/or vapors, which may be oxidized (e.g. burnt). Further, such a sensor may for instance be configured to sense ozone, particulate matter, carbon monoxide, sulfur dioxide, and nitrous oxide (e.g. odors, tobacco smoke, exhalations emitted by materials, to name but a few non-limiting examples).

The at least one sensor may for instance be an electrochemical sensor. The at least one sensor may for instance comprise an O₂-conducting electrolyte. For instance, NOx and O₂ may for instance influence a reaction rate of the O₂-conducting electrolyte. In order to determine an intensity and/or a concentration of e.g. NOx within the surrounding at which the environmental information is gathered, the reaction rate of the electrolyte may for instance be used to resolve ppm (parts per million) of NOx in relation to the surrounding consisting at least partially (e.g. between 2 % to 20 %) of Oxygen.

According to an exemplary embodiment of all aspects of the present invention, the emission of the substance is non-continuous. The emission of the substance may for instance be non-continuous e.g. in case it is timely limited. The emission of the substance may for instance be non-continuous e.g. due to the substance being volatile. The emission of the substance may for instance be non-continuous e.g. due to the amount of the substance being volatile. Thus, over time, the emission of the substance takes place, the amount of the substance is reduced due to the emission of the substance.

According to an exemplary embodiment of all aspects of the present invention, the at least one apparatus is or is part of a drone.

As used herein the term "drone" may refer to an (e.g. autonomous and/or remote controlled) UAV. Further, the term "drone" and/or the plural form of this term are used throughout herein to refer to any unmanned aerial system, in particular remotely controllable and/or operating autonomously. The drone may for instance be operated by a provider of substances, or a third party.

According to an exemplary embodiment of the first exemplary aspect of the present invention, the drone moves (e.g. flies) independent of control signals of a human operator. Such a drone within the meaning of the present invention is also referred to as an autonomous drone respectively device. For being autonomous, the drone may for instance comprise a controller, e.g. which may control (e.g. move respectively fly) the drone according to one or more controlling information. The determining of the controlling information may for instance be based, at least partially and besides the features and aspects described above of the method according to the first exemplary aspect of the present invention, on one or more sensor information, e.g. gathered by one or more sensors comprised by or being connectable to the drone. Each of the one or more sensor information may for instance be indicative of an information with respect to one or more certain parameters of the surrounding enabling the drone to (e.g. autonomously) move (e.g. navigate) according to requirements set out by the surrounding (e.g. environment in which the drone is located).

The drone may for instance comprise or be connectable to a memory, a communication interface, at least one sensor (e.g. environmental sensor; additionally an optical sensor, like a camera, to name but one non-limiting example), and a processor configured with processor-executable code to perform operations of the method according to the first exemplary aspect of the present invention. An exemplary embodiment of the apparatus performing the method according to the first exemplary aspect of the present invention may for instance comprise or include respective means for performing and/or controlling at least partially the steps of the method according to the first exemplary aspect of the present invention.

According to an exemplary embodiment of all aspects of the present invention, the drone flies according to a pre-determined or determined according to pre-defined rules path.

Such a path may for instance be represented by one or more waypoint information. Each of the one or more waypoint information may for instance be represented by one or more position information. Such a respective position information may for instance comprise latitude, longitude coordinates, and optionally altitude coordinate, or x-, y-coordinates, and optionally z-coordinate. Those coordinates may for instance be defined in relation to a venue, in which the apparatus (e.g. drone) performing and/or controlling the method according to the first exemplary aspect of the present invention is located respectively positioned. More details of the venue are described below.

The apparatus (e.g. drone) may for instance be configured according to or comprise an obstacle avoidance mechanism. For instance, the drone may fly according to the pre-determined or determined according to pre-defined rules path. There may for instance be one or more obstacles along the path (e.g. obstacles of the venue, like e.g. walls, equipment or the like, to name but a few non-limiting examples). The apparatus (e.g. drone) may for instance comprise or be connectable to at least one sensor that is configured to determine whether or not such an obstacle is along the path (e.g. based on an information gathered by a sensor (e.g. camera)). Further, the drone may for instance be configured to determine a corresponding controlling information for moving the apparatus (e.g. drone) to avoid the determined obstacle in case an obstacle is determined to be located along the path,.

According to an exemplary embodiment of all aspects of the present invention, the drone flies within a venue.

Such a venue may for instance be an outdoor area, a building (e.g. shopping mall, office building, public building, a warehouse), a station, a port, or an airport that may comprise or be polluted with an emission of the substance, to name but a few non-limiting examples.

According to an exemplary embodiment of the first exemplary aspect of the present invention, in case the gathered environmental information represents that the substance is sensed, the drone deviates from the path to enable the determining of the position information to be output.

The drone may for instance deviate from the pre-determined (e.g. defined by one or more way-points) path in order to determine the location of the emission of the substance only in case a presence of the substance is sensed. In particular, the drone may deviate from the path without requiring authorization and/or an input of a human operator. In this way, in particular by repeating the steps of the gathering of the environmental information, the determining of a location estimate information, the determining of a controlling information, and the controlling of the drone, the drone is enabled to automatically determine (e.g. detect) the location of the emission of the substance. There is no manual input or exhaustive monitoring e.g. of the venue required to be performed, e.g. by persons on site of the venue.

Further, in case the position information indicative of the location of the emission of the source is determined, the drone may for instance move back to the last point of the path, e.g. the point of the path at that the drone has deviated the path. Then, the drone may for instance continue to move along the path.

According to an exemplary embodiment of all aspects of the present invention, in case the gathered environmental information represents that the substance is sensed, the method steps are performed until the position information is determined, or an error occurs.

The method steps that are performed at least comprise or are represented by the steps of the gathering of the environmental information, the determining of a location estimate information, the determining of a controlling information, and the controlling of the drone. For instance, the drone searches for a location of an emission of a substance only in case the substance is sensed. As described above, those steps are performed until the position information is determined (e.g. location of emission is found). In case the gathered environmental information represents that no substance is sensed, there is no need for the drone to perform the steps of the gathering of the environmental information, the determining of a location estimate information, the determining of a controlling information, and the controlling of the drone, since there is no location of an emission of a substance to be found.

Further, the steps of the gathering of the environmental information, the determining of a location estimate information, the determining of a controlling information, and the controlling of the drone are not performed or stopped being performed in case an error occurs. Such an error may for instance be that e.g. an energy source (e.g. battery) of the drone is empty.

According to an exemplary embodiment of all aspects of the present invention, in case the error occurs, the method further comprises:
- determining a further controlling information indicative of requesting the drone to move to a docking station; and
- controlling the drone based at least partially on the further controlling information.

For instance, in case the error represents that state-of-charge of the energy source is low, the drone may be moving back to the docking station, wherein the drone is controlled based at least partially on the further controlling information. The drone may for instance be moved back to the docking station, e.g. for recharging the energy source.

According to an exemplary embodiment of all aspects of the present invention, in case the position information is not determined and the error occurs (e.g. state-of-charge of the energy source is low), the method further comprises:
- outputting a last position information indicative of the last location of the drone enabling another drone to continue to perform the method steps until the position information is determined.

The last position information may for instance be output to another drone, or to an apparatus that transfers the last position information to the other drone. Based on this last position information, the other drone is enabled to continue the determining of the position information that is determined to represent the location of the emission of the substance. The other drone may for instance be fully charged respectively its energy source may be fully charged in contrast to the drone. The last position information may for instance represent a location of the drone in the venue.

By assigning the determining of the position information indicative of the location of the emission of the substance (e.g. the search for the emission source) to another drone, a reliable and efficient determining (detecting) of the location of the emission of the substance is ensured. Further, the determining of the location of the emission of the substance is being made more robust since the usage of e.g. at least two drones introduces some sort of redundancy into the determining of the position information.

Additionally, the last position information may for instance comprise a plurality of position information, wherein each of the plurality of position information is indicative of a location where the emission of the substance was not reached (e.g. threshold was not met) enabling the prevention of redundant search for the location of the emission. In this way, an efficient determining of the position information indicative of the location of the emission of the substance is ensured.

The features and example embodiments of the invention described above may equally pertain to the different aspects according to the present invention.

It is to be understood that the presentation of the invention in this section is merely by way of examples and non-limiting.

Other features of the invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not drawn to scale and that they are merely intended to conceptually illustrate the structures and procedures described herein.

### BRIEF DESCRIPTION OF THE FIGURES

In the figures show:
- Fig. 1: a schematic block diagram of a system according to an exemplary aspect of the present invention;
- Fig. 2: a flow chart showing an example embodiment of a method according to the first exemplary aspect of the present invention, for instance performed by drone 130 of Fig. 1; and
- Fig. 3: a schematic block diagram of an apparatus according to an exemplary aspect of the present invention.

### DETAILED DESCRIPTION OF SOME EXAMPLE EMBODIMENTS OF THE INVENTION

The following description serves to deepen the understanding of the present invention and shall be understood to complement and be read together with the description as provided in the above SUMMARY section of this specification.

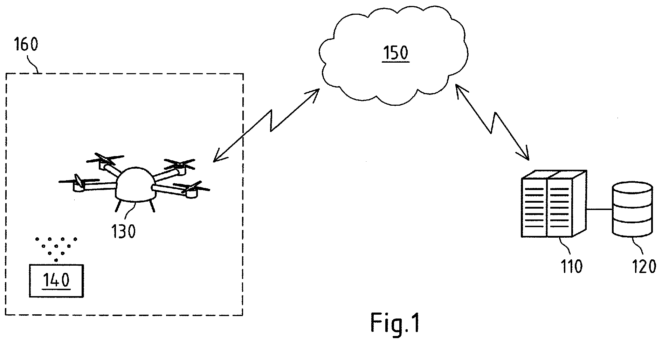

Fig. 1 shows a schematic high-level block diagram of a system 100 according to an exemplary aspect of the present invention. System 100 comprises a server 110, which may alternatively be embodied as a server cloud (e.g. a plurality of servers connected e.g. via a communication network 150, e.g. the Internet, and providing services at least partially jointly), and a drone 130, of which one exemplary realization is shown.

Further, server 110 may be connected to a database 120. The database 120 may for instance be comprised by the server 110, or may be connected to the server 120, e.g. via the communication network 150.

Server 110 and drone 130 may for instance be configured to communicate with each other, e.g. via the communication network 150.

Further, drone 130 may for instance be located in a venue 160, at present shown as a specified outdoor area. In Fig. 1, a sense-able substance 140 is a VOC, which is comprised by or located within the venue 160. An emission of the sense-able substance is schematically shown in Fig. 1 by the indicated particles shown above the sense-able substance 140.

According to embodiments of the present invention, drone 130 gathers an environmental information within the venue 160. The environmental information represents that the substance 140 is sensed. Based at least partially on the environmental information, the drone determines a location estimate information of a (e.g. possible) location of the emission of the substance 140. Then, the drone determines a controlling information and controls (e.g. moves) accordingly. In case the drone 130 has reached the location of the substance, the drone 130 determines a position information indicative of the location of the emission of the substance 140. Otherwise, the drone 130 may e.g. gather another environmental information, determine another location estimate information representing a (e.g. possible) location of the emission of the substance 140, determines another controlling information enabling the drone 130 to move (e.g. fly) towards the (e.g. possible) location, and moves according to this other (new) controlling information. In some exemplary embodiments of the present invention, this is repeated until the drone 130 has reached the substance 140. When the position information is determined, the drone 130 outputs this position information, e.g. to the server 110 via the communication network 150, e.g. for further usage. For instance, a person on site of the venue 160 may receive the position information representing that at a specific location of the venue 160 represented by the position information an emission of the substance 140 takes place, and may then perform one or more necessary steps to treat the emission of the substance 140.

According to other alternative embodiments of the present invention, in contrast to the aforementioned embodiment, the server 110 may for instance determine a location estimate information of a (e.g. possible) location of the emission of the substance 140. To enable the server 110 to determine the location estimate information, the server 110 receives an environmental information that was gathered by the drone 130 from the drone 130. Then, the determined location estimate information is output (e.g. transmitted; e.g. via the communication network 150) to the drone 130. The drone 130 determines a controlling information and controls (e.g. moves) accordingly. Alternatively, after the server 110 has determined the location estimate information, the server 110 determines the controlling information. Then, the server 110 outputs (e.g. transmitted; e.g. via the communication network 150) this determined controlling information to the drone 130. Upon reception of the controlling information from the server 110, the drone 130 controls (e.g. moves) itself accordingly. In some exemplary embodiments of the present invention, this is repeated until the drone 130 has reached the substance 140. Then, the drone 130 determines the position information, and outputs this position information, e.g. to the server 110 via the communication network 150, e.g. for further usage.

For enabling performing and/or controlling of some method steps of the method according to the first exemplary aspects of the present invention by the drone 130, and some other method steps of the method according to the first exemplary aspects of the present invention by the server 110, e.g. as described above, the drone 130 and the server 110 may use a communication connection that is established between the drone 130 and the server 110, e.g. via the communication network 150 (e.g. the Internet).

Thus, communication between the drone 130 and the server 110 may for instance take place at least partially in a wireless fashion, e.g. based on cellular radio communication or on Wireless Local Area Network (WLAN) based communication, to name but a few examples. Mobility of drone 130 is guaranteed.

In this way, the determining of a location of an emission of a substance in a reliable and efficient manner can be ensured.

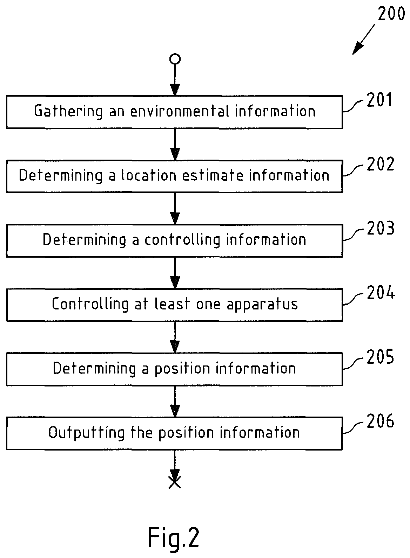

Fig. 2 is a flow chart 200 showing an example embodiment of a method according to the first exemplary aspect of the present invention. This flowchart may for instance be performed by at least one apparatus, e.g. drone 130 of Fig. 1. Alternatively, some steps of this flowchart 200 (e.g. steps 201, 204, 205 and 206) may for instance be performed by drone 130 of Fig. 1, while other steps of this flowchart 200 (e.g. steps 202, 203) may for instance be performed by server 110 of Fig. 1. In case some steps are performed by drone 130 of Fig. 1 and some other steps are performed by server 110 of Fig. 1, there maybe a communication between drone 130 of Fig. 1 and server 110 of Fig. 1 involved.

In a first step 201, environmental information is gathered, e.g. by at least one sensor comprised by or being connectable to drone 130.

In a second step 202, location estimate information is determined based at least partially on the gathered environmental information of step 201. The location estimate information may for instance be determined by the drone 130, or the location estimate information may for instance be determined by the server 110. In the latter case, prior to the determining of the location estimate information, the environmental information gathered in step 201 maybe received from the drone 130.

In a third step 203, controlling information is determined, e.g. by the drone 130, or by the server 110. The controlling information is determined based at least partially on the location estimate information of step 202.

In a fourth step 204, the drone 130 is controlled based at least partially on the controlling information of step 203. In case the controlling information is determined in step 203 by the server 110, to enable controlling of the drone 130, the drone 130 receives the controlling information from the server 110 that may for instance have transmitted the controlling information to the drone 130.

In a fifth step 205, position information is determined by the drone 130 in case the drone 130 has reached a location of an emission of the substance, e.g. as a result of the performing of the steps 201 to 204 one or more times.

In a sixth step 206, the position information determined in step 205 is output, e.g. from the drone 130 of Fig. 1 to the server 110 of Fig. 1. The position information may for instance be output via the communication connection between the drone 130 and the server 110 in case that some steps of the method according to the first exemplary aspect of the present invention are performed by the drone 130, while other steps of this method are performed by the server 110. Alternatively or additionally, the position information may for instance be output by the drone 130 via one or more broadcasts.

The exemplary flowchart 200 of Fig. 2 may for instance comprise one or more further features described above, for instance whether or not the location of the emission of the substance is (already) reached by the drone 130, and depending on the outcome of this check, e.g. repeating the steps 201 to 204, or to continue performing the flowchart 200 with the steps 205 and 206.

Furthermore, the step 201 (gathering of an environmental information) and the step 204 (controlling the drone) may for instance be performed either serial (as shown in Fig. 2) or in parallel (e.g. in case steps 201 to 204 are repeated since e.g. the drone 130 has not yet reached the location of the emission of the substance).

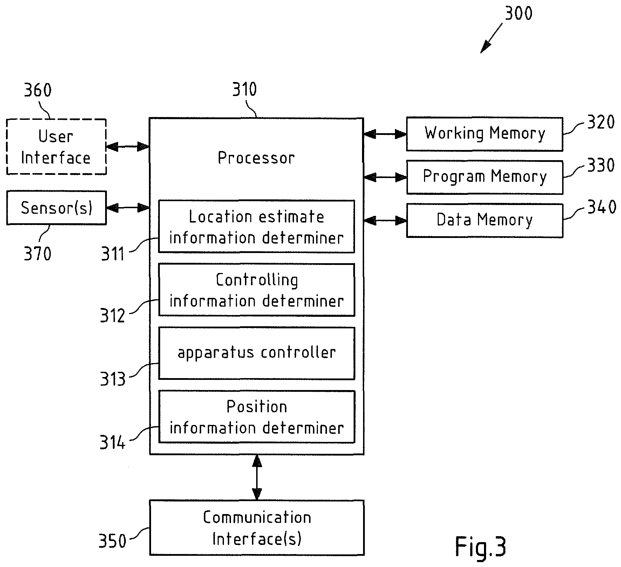

Fig. 3 is a schematic block diagram of an apparatus 300 according to an exemplary aspect of the present invention, which may for instance represent the drone 130 of Fig. 1, and/or the server 110 of Fig. 1. In case apparatus 300 represents server 110 of Fig. 1, blocks 313, 314, and 370 are not comprised by such an apparatus.

Apparatus 300 comprises a processor 310, working memory 320, program memory 330, data memory 340, communication interface(s) 350, an optional user interface 360 and (an) optional sensor(s) 370.

Apparatus 300 may for instance be configured to perform and/or control or comprise respective means (at least one of 310 to 370) for performing and/or controlling the method according to the first exemplary aspect of the present invention. Apparatus 300 may as well constitute an apparatus comprising at least one processor (310) and at least one memory (320) including computer program code, the at least one memory and the computer program code configured to, with the at least one processor, cause an apparatus, e.g. apparatus 300 at least to perform and/or control the method according to the first exemplary aspect of the present invention.

Processor 310 may for instance comprise location estimate information determiner 311 as a functional and/or structural unit. Location estimate information determiner 311 may for instance be configured to determine a location estimate information (see step 202 of Fig. 2). Processor 310 may for instance comprise controlling information determiner 312 as a functional and/or structural unit. Controlling information determiner 312 may for instance be configured to determine a controlling information based at least partially on a location estimate information (see step 203 of Fig. 2). Processor 310 may for instance comprise an apparatus controller 313 as a functional and/or structural unit. Apparatus controller 313 may for instance be configured to control the apparatus 300, e.g. move (e.g. fly) the apparatus in a certain direction, change altitude, roll and/or tilt the apparatus 300, and/or enable the apparatus to autonomously move to a location represented by the location estimate information, to name but a few non-limiting examples (see step 204 of Fig. 2). Processor 310 may for instance comprise position information determiner 314 as a functional and/or structural unit. Position information determiner 314 may for instance be configured to determine a position information in case the apparatus 300 has reached a location of an emission of a substance (see step 205 of Fig. 2).

Processor 310 may for instance further control the memories 320 to 340, the communication interface(s) 350, the optional user interface 360 and the optional sensor(s) 370.

Processor 310 may for instance execute computer program code stored in program memory 330, which may for instance represent a computer readable storage medium comprising program code that, when executed by processor 310, causes the processor 310 to perform the method according to the first exemplary aspect of the present invention.

Processor 310 (and also any other processor mentioned in this specification) may be a processor of any suitable type. Processor 310 may comprise but is not limited to one or more microprocessor(s), one or more processor(s) with accompanying one or more digital signal processor(s), one or more processor(s) without accompanying digital signal processor(s), one or more special-purpose computer chips, one or more field-programmable gate array(s) (FPGA(s)), one or more controller(s), one or more application-specific integrated circuit(s) (ASIC(s)), or one or more computer(s). The relevant structure/hardware has been programmed in such a way to carry out the described function. Processor 310 may for instance be an application processor that runs an operating system.

Program memory 330 may also be included into processor 310. This memory may for instance be fixedly connected to processor 310, or be at least partially removable from processor 310, for instance in the form of a memory card or stick. Program memory 330 may for instance be non-volatile memory. It may for instance be a FLASH memory (or a part thereof), any of a ROM, PROM, EPROM and EEPROM memory (or a part thereof) or a hard disc (or a part thereof), to name but a few examples. Program memory 330 may also comprise an operating system for processor 310. Program memory 330 may also comprise a firmware for apparatus 300.

Apparatus 300 comprises a working memory 320, for instance in the form of a volatile memory. It may for instance be a Random Access Memory (RAM) or Dynamic RAM (DRAM), to give but a few non-limiting examples. It may for instance be used by processor 310 when executing an operating system and/or computer program.

Data memory 340 may for instance be a non-volatile memory. It may for instance be a FLASH memory (or a part thereof), any of a ROM, PROM, EPROM and EEPROM memory (or a part thereof) or a hard disc (or a part thereof), to name but a few examples. Data memory 340 may for instance store one or more pieces of location estimate information, one or more pieces of controlling information, one or more pieces of position information, to name but a few non-limiting examples. Data memory 340 may for instance store further pieces of information as described above, and/or one or more pieces of interim result information that may occur e.g. when performing and/or controlling example embodiments of the method according to the first exemplary aspect of the present invention.

Communication interface(s) 350 enable apparatus 300 to communicate with other entities, e.g. with server 110 of Fig. 1 in case apparatus 300 is represented by drone 130 of Fig. 1; or with drone 130 of Fig. 1 in case apparatus 300 is represented by server 110 of Fig. 1. The communication interface(s) 350 may for instance comprise a wireless interface, e.g. a cellular radio communication interface and/or a WLAN, BT and/or BLE interface, for instance to communicate with entities, e.g. via communication network 150 of Fig. 1 (e.g. the Internet).

User interface 360 is optional and may comprise a display for displaying information to a user and/or an input device (e.g. a keyboard, keypad, touchpad, mouse, etc.) for receiving information from a user.

Sensor(s) 370 may for instance comprise an environmental sensor (e.g. air pollution sensor) and/or an optical sensor (e.g. a camera), to name but a few non-limiting examples. In particular, sensor(s) may for instance be configured at least to sense VOC, NOx, and/or SOx.

Some or all of the components of the apparatus 300 may for instance be connected via a bus. Some or all of the components of the apparatus 300 may for instance be combined into one or more modules.

In the present specification, any presented connection in the described embodiments is to be understood in a way that the involved components are operationally coupled. Thus, the connections can be direct or indirect with any number or combination of intervening elements, and there may be merely a functional relationship between the components.

Moreover, any of the methods, processes and actions described or illustrated herein may be implemented using executable instructions in a general-purpose or special-purpose processor and stored on a computer-readable storage medium (e.g., disk, memory, or the like) to be executed by such a processor. References to a 'computer-readable storage medium' should be understood to encompass specialized circuits such as FPGAs, ASICs, signal processing devices, and other devices.

The expression "A and/or B" is considered to comprise any one of the following three scenarios: (i) A, (ii) B, (iii) A and B. Furthermore, the article "a" is not to be understood as "one", i.e. use of the expression "an element" does not preclude that also further elements are present. The term "comprising" is to be understood in an open sense, i.e. in a way that an object that "comprises an element A" may also comprise further elements in addition to element A.

It will be understood that all presented embodiments are only exemplary, and that any feature presented for a particular example embodiment may be used with any aspect of the invention on its own or in combination with any feature presented for the same or another particular example embodiment and/or in combination with any other feature not mentioned. In particular, the example embodiments presented in this specification shall also be understood to be disclosed in all possible combinations with each other, as far as it is technically reasonable and the example embodiments are not alternatives with respect to each other. It will further be understood that any feature presented for an example embodiment in a particular category (method/apparatus/computer program/system) may also be used in a corresponding manner in an example embodiment of any other category. It should also be understood that presence of a feature in the presented example embodiments shall not necessarily mean that this feature forms an essential feature of the invention and cannot be omitted or substituted.

The statement of a feature comprises at least one of the subsequently enumerated features is not mandatory in the way that the feature comprises all subsequently enumerated features, or at least one feature of the plurality of the subsequently enumerated features. Also, a selection of the enumerated features in any combination or a selection of only one of the enumerated features is possible. The specific combination of all subsequently enumerated features may as well be considered. Also, a plurality of only one of the enumerated features may be possible.

The sequence of all method steps presented above is not mandatory, also alternative sequences may be possible. Nevertheless, the specific sequence of method steps exemplarily shown in the figures shall be considered as one possible sequence of method steps for the respective embodiment described by the respective figure.

The invention has been described above by means of example embodiments. It should be noted that there are alternative ways and variations which are obvious to a skilled person in the art and can be implemented without deviating from the scope of the appended claims.

## Claims

1. A method, performed by at least one apparatus, comprising:
- gathering an environmental information indicative of a sense-able substance;
- determining a location estimate information indicative of an estimation of a location of an emission of the substance;
- determining a controlling information indicative of a movement of the at least one apparatus towards the estimation of the location of the sense-able substance, wherein the controlling information is determined based at least partially on the location estimate information;
- controlling the at least one apparatus towards the source of the sense-able substance based at least partially on the controlling information;
- determining a position information indicative of the location of the emission of the substance in case the location of the emission of the substance is reached; and
- outputting the position information.

2. The method according to claim 1, wherein the location of the substance is reached in case the environmental information exceeds a pre-determined or determined according to pre-defined rules threshold.

3. The method according to claim 1 or claim 2, wherein the steps of the gathering of the environmental information, the determining of a location estimate information, the determining of a controlling information, and the controlling of the at least one apparatus are repeated until the location of the substance is reached.

4. The method according to any of the preceding claims, further comprising:
- treatment of the substance based at least partially on the output position information.

5. The method according to any of the preceding claims, wherein the environmental information is gathered by at least one sensor that is configured at least to sense one or more volatile organic compounds (VOC), mono-nitrogen oxides (NOx), and/or sulfur oxides (SOx).

6. The method according to any of the preceding claims, wherein the emission of the substance is non-continuous.

7. The method according to any of the preceding claims, wherein the at least one apparatus is or is part of a drone.

8. The method according to claim 7, wherein the drone flies according to a pre-determined or determined according to pre-defined rules path.

9. The method according to claim 7 or claim 8, wherein the drone flies within a venue.

10. The method according to claim 8 or claim 9, wherein in case the gathered environmental information represents that the substance is sensed, the drone deviates from the path to enable the determining of the position information to be output.

11. The method according to any of the claims 7 to 10, wherein in case the gathered environmental information represents that the substance is sensed, the method steps are performed until the position information is determined, or an error occurs.

12. The method according to claim 11, in case the error occurs, the method further comprises:
- determining a further controlling information indicative of requesting the drone to move to a docking station; and
- controlling the drone based at least partially on the further controlling information.

13. The method according to claim 11 or claim 12, wherein in case the position information is not determined and the error occurs, the method further comprises:
- outputting a last position information indicative of the last location of the drone enabling another drone to continue to perform the method steps until the position information is determined.

14. An apparatus configured to perform and/or control or comprising respective means for performing and/or controlling the method of any of the claims 1 to 13.

15. A system, comprising:
- at least one apparatus; and
- a server or a server-cloud, wherein the at least one apparatus and the server are configured to perform and/or control the method according to any of the claims 1 to 13.
